# DEMANDE DE BREVET EUROPEEN

(11) **EP 1 552 810 A1**
(43) Date de publication de la demande: **13.07.2005**
(21) Numéro de dépôt: 04293028.9
(22) Date de dépôt: 17.12.2004
(51) Int. Cl.: A61K 7/075

(54) **Compositions cosmétiques détergentes comprenant une silicone et un polymère de haut poids moléculaire et utilisation de ces dernières**

(30) Priorité: 07.01.2004 FR 0450031; 07.01.2004 FR 0450039
(71) Demandeur: L'OREAL, 75008 Paris (FR)
(72) Inventeur: Samain, Henri, 91570 Bièvres (FR); Fack, Géraldine, 92300 Levallois (FR); Giroud, Franck, 92110 Clichy (FR); Paul, Laurence, 95320 Saint Leu La Forêt (FR)
(74) Mandataire: Le Blainvaux Bellegarde, Françoise

(57) **Abrégé**

L'invention concerne de nouvelles compositions de lavage notamment capillaires, comprenant, dans un milieu cosmétiquement acceptable, au moins une silicone non aminée ayant une viscosité inférieure à 100.000 cSt, au moins un tensioactif détergent anionique, non ionique ou amphotère et au moins un polymère filant.

Ces compositions possèdent un effet coiffant amélioré.

## Description

La présente invention concerne de nouvelles compositions cosmétiques à propriétés améliorées destinées simultanément au nettoyage, au conditionnement et au coiffage des matières kératiniques notamment des cheveux, et comprenant, dans un support cosmétiquement acceptable, une base lavante constituée de tensio-actifs à pouvoir détergent dans laquelle sont également présents des silicones non aminées en association avec des polymères filants de haut poids moléculaire particuliers. L'invention concerne aussi l'utilisation desdites compositions dans l'application cosmétique susmentionnée.

Pour le nettoyage et/ou le lavage des cheveux, l'utilisation de compositions capillaires détergentes (ou shampooings) à base essentiellement d'agents tensio-actifs classiques de type notamment anioniques, non-ioniques et/ou amphothères, mais plus particulièrement de type anioniques, est courante. Ces compositions sont appliquées sur cheveux mouillés et la mousse générée par massage ou friction avec les mains permet, après rinçage à l'eau, l'élimination des diverses salissures initialement présentes sur les cheveux.

Ces compositions de base possèdent certes un bon pouvoir lavant, mais les propriétés cosmétiques intrinsèques qui leur sont attachées restent toutefois assez faibles, notamment en raison du fait que le caractère relativement agressif d'un tel traitement de nettoyage peut entrainer à la longue sur la fibre capillaire des dommages plus ou moins marqués liés en particulier à l'élimination progressive des lipides ou protéines contenues dans ou à la surface de cette dernière.

Aussi, pour améliorer les propriétés cosmétiques des compositions détergentes ci-dessus, et plus particulièrement de celles qui sont appelées à être appliquées sur des cheveux sensibilisés (i.e. des cheveux qui se trouvent abimés ou fragilisés notamment sous l'action chimique des agents atmosphériques et/ou de traitements capillaires tels que permanentes, teintures ou décolorations), il est maintenant usuel d'introduire dans ces dernières des agents cosmétiques complémentaires dits agents conditionneurs destinés principalement à réparer ou limiter les effets néfastes ou indésirables induits par les différents traitements ou agressions que subissent, de manière plus ou moins répétés, les fibres capillaires. Ces agents conditionneurs peuvent bien entendu également améliorer le comportement cosmétique des cheveux naturels.

Les agents conditionneurs les plus couramment utilisés à ce jour dans des shampooings sont les polymères cationiques, qui confèrent en effet aux cheveux lavés, secs ou mouillés, une facilité de démélage, une douceur et un lissage nettement accrus par rapport à ce qui peut être obtenu avec les compositions nettoyantes correspondantes qui en sont exemptes.

Par ailleurs, depuis quelque temps, on cherche à obtenir des shampooings conditionneurs qui soient capables d'apporter aux cheveux lavés non seulement les propriétés cosmétiques mentionnées ci-avant mais aussi, à un degré plus ou moins poussé, des propriétés de coiffage, de volume, de mise en forme et de tenue. Ces derniers shampooings lavants à propriétés cosmétiques générales améliorées sont souvent dénommés, par simplicité, "shampooings à effets coiffants", expression qui sera d'ailleurs reprise dans la suite de l'exposé.

Toutefois, et malgré les progrès réalisés récemment dans le domaine des shampooings à effets coiffants, ces derniers ne donnent pas vraiment complètement satisfaction, de sorte qu'un fort besoin existe encore actuellement quant à pouvoir disposer de nouveaux produits présentant, au niveau de l'une ou de plusieurs des propriétés cosmétiques évoqués ci-avant, de meilleures performances. En particulier, il est le plus souvent nécessaire d'utiliser un produit de coiffage après le shampooing pour donner une forme à la chevelure et pour fixer la coiffure.

La présente invention vise à la satisfaction d'un tel besoin.

Ainsi, à la suite d'importantes recherches menées sur la question, il a maintenant été trouvé par la Demanderesse, de façon totalement inattendue et surprenante, qu'en associant des silicones non aminées de viscosité particulière, tels que définis ci-après, avec certains polymères présentant un pouvoir filant particulier dans des compositions détergentes, il est possible d'améliorer de manière substantielle et significative les propriétés de coiffage et de maintien, et ceci tout en conservant leur bon pouvoir lavant intrinsèque et leurs propriétés cosmétiques.

Notamment, ces compositions permettent d'obtenir un très bon maintien et un certain volume de la chevelure, c'est-à-dire un effet coiffant similaire à celui obtenu avec un gel de coiffage de fixation utilisé après un shampooing. On constate par ailleurs que les fibres kératiniques sont renforcées (durcies).

Toutes ces découvertes sont à la base de la présente invention.

Ainsi, selon la présente invention, il est maintenant proposé de nouvelles compositions de lavage des matières kératiniques, en particulier des cheveux comprenant, dans un milieu aqueux cosmétiquement acceptable, au moins une silicone non aminée ayant une viscosité inférieure à 100.000 cst (mm²/s), au moins un tensioactif détergent anionique, non ionique ou amphotère et au moins un polymère présentant un pouvoir filant supérieur à 5 cm.

L'invention a également pour objet l'utilisation en cosmétique des compositions ci-dessus pour le nettoyage, le conditionnement, le soin et le coiffage des matières kératiniques notamment les cheveux et les cils.

Un autre objet de l'invention consiste en un procédé de traitement cosmétique des matières kératiniques notamment des fibres kératiniques mettant en oeuvre la composition selon l'invention et plus particulièrement les cheveux et les cils.

L'invention a encore pour objet une utilisation de la composition selon l'invention comme shampoing.

Mais d'autres caractéristiques, aspects et avantages de l'invention apparaîtront encore plus clairement à la lecture de la description qui va suivre ainsi que des exemples concrets, mais nullement limitatifs, destinés à l'illustrer.

Le pouvoir filant d'un polymère correspond à la longueur du fil de polymère obtenu à la rupture dudit fil selon le mode opératoire défini ci-dessous.

Le pouvoir filant des polymères utilisables selon l'invention est celui qui est mesurée pour une composition contenant (% en poids):

| | |
|---|---|
| Lauryléthersulfate de sodium oxyéthyléné à 2,2 moles | |
| d'oxyde d'éthylène | 12,5% |
| Cocoamidopropylbétaïne | 2,5 % |
| Polymère à tester | 1 % |
| Eau qsp | 100%. |

La mesure du pouvoir filant se fait à l'aide d'un analyseur de texture TA-TX2 (Rhéo/stable Micro Systems)

La mesure se fait après compression du produit :
- Déplacement du disque (cylindre 35 mm en aluminium) à la vitesse de 2,5 mm/s et détection de la force de résistance à la compression
- Pénétration dans le produit à la même vitesse sur une profondeur de 10 mm
- Retrait de la sonde à la vitesse de 2.5 mm/s
- Mesure du déplacement de la sonde et détection de la rupture du fil de produit

Selon la présente invention, les polymères présentant un pouvoir filant supérieur à 5 cm seront aussi appelés polymères filants.

Les polymères présentant un pouvoir filant supérieur à 5 cm sont notamment soit (a1) une dispersion de particules d'un polymère cationique hydrosoluble ayant une masse moléculaire moyenne en poids supérieure à 10⁶ dans une solution aqueuse saline, obtenu par polymérisation radicalaire hétérogène de monomères hydrosolubles avec précipitation du polymère formé, ou soit (a2) une solution aqueuse d'un polymère cationique hydrosoluble ayant une masse moléculaire moyenne en poids supérieure à 10⁶, obtenu par polymérisation radicalaire hétérogène de monomères hydrosolubles avec précipitation du polymère formé.

Par polymère cationique, on comprend les polymères comprenant des monomères cationiques et éventuellement des monomères non-ioniques.

Le polymère cationique hydrosoluble de haut poids moléculaire est un polyélectrolyte polymérisé à partir d'au moins un monomère cationique de formule I).

Comme indiqué ci-dessus, la synthèse des polymères hydrosolubles de haut poids moléculaire, utilisés dans la présente invention, se fait par polymérisation radicalaire hétérogène de monomères hydrosolubles comportant au moins une insaturation éthylénique. La polymérisation se fait dans une solution aqueuse d'un électrolyte minéral (sel) ayant une force ionique suffisante pour provoquer la précipitation du polymère formé dès que celui-ci atteint une certaine masse moléculaire. Cette technique de polymérisation permet ainsi, grâce au phénomène bien connu de relargage par des sels (*salting-out*)*,* la préparation de dispersions aqueuses salines de particules de polymères hydrosolubles. Les polymères ainsi synthétisés se distinguent par une masse moléculaire moyenne en poids élevée, généralement supérieure à 10⁶.

La technique de polymérisation radicalaire hétérogène en milieu aqueux avec précipitation du polymère formé est décrite par exemple dans le brevet US 4 929 655, dans la demande de brevet EP 0 943 628 ou encore dans la demande internationale WO 02/34796.

Pour garantir la stabilité des dispersions de particules de polymère pendant la synthèse et au cours du stockage, il est généralement indispensable de réaliser la polymérisation en présence d'un agent dispersant. Cet agent dispersant est de préférence un polyélectrolyte qui, contrairement au polymère de haut poids moléculaire utilisé dans l'invention, est soluble dans le milieu aqueux de polymérisation de force ionique élevée.

Ce polyélectrolyte dispersant a de préférence une charge identique à celle du polymère synthétisé, autrement dit pour la synthèse de polyélectrolytes cationiques, on utilise généralement un polyélectrolyte dispersant cationique.

On peut citer à titre d'agents dispersants préférés, les polyélectrolytes cationiques obtenus par polymérisation de 50 à 100 % en moles d'au moins un monomère cationique choisi parmi les sels, de préférence les chlorhydrates ou sulfates, de (méth)acrylate de diméthylaminoéthyle, de N-diméthylaminopropyl(méth)acrylamide ou de di(méth)allylamine, le chlorure de (méth)acryloyloxyéthyltriméthylammonium, le chlorure de (méth)acrylamidopropyltriméthylammonium et le chlorure de diméthyldiallylammonium, et de 50 à 0 % en moles d'acrylamide. On peut également utiliser une polyamine telle qu'une polyalkylèneamine.

L'agent dispersant est utilisé de préférence à raison de 1 à 10 % en poids, rapporté au poids total des monomères à polymériser.

La solution aqueuse saline qui sert de milieu de synthèse et de dispersion au polymère hydrosoluble de haut poids moléculaire est une solution d'un ou de plusieurs sels minéraux choisis de préférence parmi les sels anioniques divalents. On peut citer en tant que sels anioniques par exemple le sulfate d'ammonium, l'hydrogénosulfate d'ammonium, le chlorure d'ammonium, le sulfate de sodium, l'hydrogénosulfate de sodium, le sulfate de magnésium, l'hydrogénosulfate de magnésium, le sulfate d'aluminium et l'hydrogénosulfate d'aluminium. Le sulfate d'ammonium et le sulfate de sodium sont particulièrement préférés.

La concentration de ce(s) sel(s) doit de préférence être suffisante pour induire la précipitation du polymère hydrosoluble formé dans le milieu de polymérisation et peut aller jusqu'à la concentration de saturation de chaque sel. Pour obtenir une telle précipitation, la concentration en sel est de préférence au moins égale à 10 % en poids, de préférence supérieure à 15% en poids et généralement inférieure à 50% en poids par rapport au poids total de la solution ou de la dispersion de polymère. La solution aqueuse saline peut contenir en outre des sels monovalents tels que le chlorure de sodium et le chlorure d'ammonium.

La polymérisation radicalaire hétérogène en milieu aqueux telle que décrite ci-dessus s'accompagne le plus souvent d'une augmentation importante de la viscosité du milieu réactionnel, qui se traduit par des difficultés d'agitation, un défaut d'homogénéité du milieu réactionnel et un élargissement de la granulométrie des particules de polymère formé. Pour empêcher une telle augmentation de la viscosité, il a été proposé, dans la demande de brevet européen EP 0 943 628, d'ajouter au milieu de polymérisation au moins un agent empêchant l'augmentation de la viscosité du milieu réactionnel en cours de polymérisation.

Les polymères hydrosolubles de haut poids moléculaire utilisés dans la présente invention sont de préférence préparés en présence d'un tel agent empêchant l'augmentation de la viscosité.

Ces agents empêchant l'augmentation de la viscosité du milieu réactionnel sont choisis par exemple parmi
(1) les acides polycarboxyliques et leurs sels,
(2) les polyphénols,
(3) les composés cycliques contenant un groupe hydroxy et un groupe carboxy, ou leurs sels,
(4) l'acide gluconique ou ses sels,
(5) les produits réactionnels obtenus par réaction d'une méthoxyhydroquinone et/ou d'un monomère cationique (méth)acrylique avec un composé qui génère des radicaux, sous une atmosphère oxydante,
(6) les produits réactionnels obtenus par réaction d'un polymère cationique (méth)acrylique avec un composé qui génère des radicaux, sous une atmosphère oxydante,
(7) les produits réactionnels obtenus par réaction d'un polymère cationique (méth)acrylique avec un agent oxydant,
et leurs mélanges.

L'addition d'au moins un agent empêchant l'augmentation de la viscosité tel que décrit ci-dessus permet d'effectuer la polymérisation des monomères hydrosolubles décrits ci-dessus avec un agitateur à faible puissance tout en évitant la formation de particules grossières. Les agents empêchant l'augmentation de la viscosité sont de préférence solubles dans le milieu réactionnel aqueux.

Comme exemples de composés (A), on peut citer l'acide oxalique, l'acide adipique, l'acide tartrique, l'acide malique, l'acide phtalique et leurs sels.

Comme exemples de composés (B), on peut notamment citer le résorcinol et le pyrogallol.

Comme exemples de composés (C), on peut citer l'acide m-hydroxybenzoïque, l'acide p-hydroxybenzoïque, l'acide salicylique, l'acide gallique, l'acide tannique et les sels de ces acides.

Comme exemples de composés (D), on peut citer le gluconate de sodium, le gluconate de potassium, le gluconate d'ammonium et différents sels aminés de l'acide gluconique.

Comme exemples de composés (E), on peut citer ceux obtenus par réaction d'un composé qui génère des radicaux, sous un courant de gaz oxygéné, dans une solution contenant de la méthoxyhydroquinone et/ou un monomère cationique (méth)acrylique. Le composé qui génère des radicaux peut être un amorceur couramment utilisé pour la polymérisation radicalaire. On peut citer par exemple les amorceurs azoïques hydrosolubles tels que le chlorhydrate de 2,2'-azo-bis(2-amidinopropane) vendu par exemple sous la dénomination V-50 par la société Wako Chemical Industries, ou le chlorhydrate de 2,2'-azo-bis[2-(2-imidazoline-2-yl)propane] vendu par exemple sous la dénomination commerciale VA-044 par la société Wako Chemical Industries, ou un amorceur du groupe des oxydoréducteurs hydrosolubles tels que l'association persulfate d'ammonium/hydrogénosulfite de sodium.

On peut obtenir un agent empêchant l'augmentation de la viscosité (F) par réaction d'un amorceur radicalaire, sous atmosphère oxygénée, avec un agent dispersant selon l'invention. L'amorceur de polymérisation peut être un amorceur azoïque hydrosoluble ou un oxydoréducteur hydrosoluble tels que décrits ci-dessus.

Les composés (G) peuvent être obtenus sous la forme de polymères oxydés de faible masse moléculaire par oxydation d'un agent dispersant cationique selon l'invention obtenu par polymérisation d'un monomère cationique (méth)acrylique, utilisant en tant qu'agent oxydant du peroxyde d'hydrogène ou un halogène.

Comme monomères cationiques (méth)acryliques utilisés pour la préparation des agents empêchant l'augmentation de la viscosité (E), (F) et (G), on peut citer par exemple le chlorhydrate ou le sulfate de (méth)acrylate de diméthylaminoéthyle, le chlorure de (méth)acryloyloxyéthyltriméthyl-ammonium, le chlorure de (méth)acryloyloxyéthyldiméthylbenzylammonium, le chlorhydrate ou le sulfate dérivé du N-diméthylaminopropyl(meth)-acrylamide, le chlorure de (méth)acrylamidopropyltriméthylammonium, le chlorure ou le sulfate de (méth)acrylate de diméthylaminohydroxypropyle, le chlorure de (méth)acryloyloxyhydroxypropyltriméthylammonium, le chlorure de (méth)acryloyloxyhydroxypropyldiméthylbenzylammonium.

Ces agents empêchant l'augmentation de la viscosité (A) à (G) peuvent être utilisés seuls ou en mélange, en une quantité comprise de préférence entre 10 ppm et 10 000 ppm par rapport au poids total de la solution réactionnelle.

Les monomères hydrosolubles polymérisés par polymérisation radicalaire hétérogène en vue de l'obtention des polymères cationiques hydrosolubles de haut poids moléculaire sont des monomères comportant au moins une double liaison éthylénique, par exemple une double liaison vinylique, acrylique ou allylique. Ils peuvent être cationiques, ou non-ioniques et être utilisés en mélange.

A titre de monomères non-ioniques hydrosolubles, on peut citer l'acrylamide, le méthacrylamide, le N-vinylformamide, le N-vinylacétonamide, l'acrylate d'hydroxypropyle et le méthacrylate d'hydroxypropyle.

Les monomères cationiques hydrosolubles sont de préférence choisis parmi les sels de di(alkyle en C₁₋₄)-diallylammonium et les composés de formule (I) 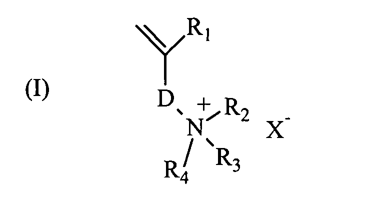 dans laquelle
R₁ représente un atome d'hydrogène ou un groupe méthyle,
R₂ et R₃, identiques ou différents, représentent chacun un atome d'hydrogène ou un groupe alkyle en C₁₋₄ linéaire ou ramifié,
R₄ représente un atome d'hydrogène, un groupe alkyle en C₁₋₄ linéaire ou ramifié ou un groupe aryle,
D représente le motif suivant 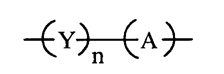 dans lequel Y représente une fonction amide (-CO-NH-), ester (-O-CO- ou -COO-), uréthane (-O-CO-NH-) ou urée (-NH-CO-NH-),
A représente un groupe alkyène en C₁₋₁₀ linéaire, ramifié ou cyclique, pouvant être substitué ou interrompu par un cycle aromatique ou hétéroaromatique divalent, ou pouvant être interrompu par un hétéroatome choisi parmi O, N, S et P, et pouvant comporter une fonction cétone, amide, ester, uréthane ou urée,
n vaut 0 ou 1, et
X⁻ représente un contre-ion anionique tel qu'un ion chlorure ou sulfate.

A titre d'exemples de monomères cationiques hydrosolubles, on peut citer le chlorhydrate ou le sulfate de (méth)acrylate de diméthylaminoéthyle, le chlorure de (méth)acryloyloxyéthyltriméthyl-ammonium, le chlorure de (méth)acryloyloxyéthyldiméthylbenzylammonium, le chlorhydrate ou sulfate de N-diméthylaminopropyl-(méth)acrylamide, le chlorure de (méth)acrylamidopropyltriméthylammonium, le chlorure de (méth)acrylamidopropyldiméthylbenzylammonium, le chlorhydrate ou sulfate de (méth)acrylate de diméthylaminohydroxypropyle, le chlorure de (méth)acryloyloxyhydroxypropyltriméthylammonium, le chlorure de (méth)acryloyloxyhydroxypropyldiméthylbenzylammonium et le chlorure de diméthyldiallylammonium.

Dans un mode de réalisation préféré de la présente invention, le polymère hydrosoluble de haut poids moléculaire est obtenu par polymérisation radicalaire hétérogène d'un mélange de monomères comprenant, de 0 à 95,5 % en moles d'acrylamide et de 4,5 à 100 % en moles d'au moins un monomère cationique de formule (I).

Selon un mode de réalisation particulièrement préféré, les polymères hydrosolubles sont obtenus par polymérisation d'un mélange de monomères constitué d'acrylamide et d'un monomère cationique de formule (I), dans lequel le nombre de moles de monomère cationique de formule (I) est supérieur au nombre de moles d'acrylamide.

Des polymères cationiques hydrosolubles particulièrement préférés sont par exemple ceux polymérisés à partir de mélanges de monomères constitués respectivement
1. de 10 % en moles de chlorure d'acryloyloxyéthyldiméthylbenzylammonium et de 90 % en moles d'acrylamide.
2. de 30 % en moles de chlorure d'acryloyloxytriméthylammonium, de 50 % en moles de chlorure d'acryloyloxyéthyldiméthybenzylammonium et de 20 % en moles d'acrylamide.
3. de 10 % en moles de chlorure d'acryloyloxyéthyltriméthylammonium et de 90 % en moles d'acrylamide.
4. de 30 % en moles de chlorure de diallyldiméthylammonium et de 70 % en moles d'acrylamide.

Les polymères hydrosolubles utilisés dans la présente invention ont une masse moléculaire moyenne en poids supérieure à 1 000 000, de préférence allant de 1 000 000 et 50 000 000 et plus particulièrement allant de 1 000 000 et 20 000 000. Cette masse moléculaire moyenne en poids est déterminée par la méthode RSV (*Reduced Specific Viscosity*) telle que définie dans « Principles of Polymer Chemistry », Cornell University Press, Ithaca, NY, 1953, Chapitre VII intitulé « Determination of Molecular Weight », pages 266 - 316.

La concentration de la dispersion ou de la solution de polymère hydrosoluble de haut poids moléculaire est choisie de préférence de manière à ce que la concentration du polymère hydrosoluble aille de 0,01 à 10 % en poids, de préférence de 0,05 à 5 % en poids par rapport au poids total de la composition finale.

La concentration du polymère filant selon l'invention va de préférence de 0,01 à 10 % en poids, de préférence de 0,05 à 5 % en poids par rapport au poids total de la composition finale.

Le ou les tensioactifs détergents sont choisis, seuls ou en mélanges, au sein des tensioactifs anioniques, amphotères, non ioniques et zwittérioniques.

Ainsi, selon l'invention, les tensioactifs détergents représentent de 4 % à 50 % en poids, de préférence de 6 % à 30 % en poids, et encore plus préférentiellement de 8 % à 25 % en poids, du poids total de la composition finale.

Les tensioactifs convenant à la mise en oeuvre de la présente invention sont notamment les suivants :

### (i) Tensioactif(s) anionique(s) :

Leur nature ne revêt pas, dans le cadre de la présente invention, de caractère véritablement critique.

Ainsi, à titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides éthers carboxyliques polyoxyalkylénés et leurs sels, en particulier ceux comportant de 2 à 50 groupements oxyde d'éthylène, et leurs mélanges. Les tensioactifs anioniques du type acides ou sels d'éthers carboxyliques polyoxyalkylénés sont en particulier ceux qui répondent à la formule (1) suivante : 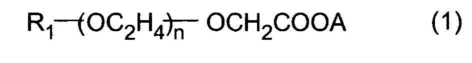 dans laquelle :
R₁ désigne un groupement alkyle alkylamido ou alkaryle, et n est un nombre entier ou décimal (valeur moyenne) pouvant varier de 2 à 24 et de préférence de 3 à 10, le radical alkyle ayant entre 6 et 20 atomes de carbone environ, et aryle désignant de préférence phényle,
A désigne H, ammonium, Na, K, Li, Mg ou un reste monoéthanolamine ou triéthanolamine. On peut également utiliser des mélanges de composés de formule (1) en particulier des mélanges dans lesquels les groupements R₁ sont différents.

Des composés de formule (1) sont vendus par exemple par la Société CHEM Y sous les dénominations AKYPO (NP40, NP70, OP40, OP80, RLM25, RLM38, RLMQ 38 NV, RLM 45, RLM 45 NV, RLM 100, RLM 100 NV, RO 20, RO 90, RCS 60, RS 60, RS 100, RO 50) ou par la Société SANDOZ sous les dénominations SANDOPAN (DTC Acid, DTC).

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools gras polyéthoxylés, polypropoxylés ou polyglycérolés, les alpha-diols gras polyéthoxylés, polypropoxylés ou polyglycérolés, les alkylphénols gras polyéthoxylés, polypropoxylés ou polyglycérolés et les acides gras polyéthoxylés, polypropoxylés ou polyglycérolés, tous ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensio-actifs non-ioniques rentrant particulièrement bien dans le cadre de la présente invention.

### (iii) Tensioactif(s) amphotère(s) ou zwittérionique(s) :

Les agents tensioactifs amphotères ou zwitterioniques, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

R₂ -CONHCH₂CH₂ -N⁺(R₃)(R₄)(CH₂COO⁻) (2)

dans laquelle : R₂ désigne un radical alkyle d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle
et

R₂'-CONHCH₂CH₂-N(B)(C) (3)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO₃H
R₂' désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

A titre d'exemple on peut citer le cocoamphocarboxyglycinate vendu sous la dénomination commerciale MIRANOL C2M concentré par la Société MIRANOL.

Dans les compositions conformes à l'invention, on peut utiliser des mélanges d'agents tensioactifs et en particulier des mélanges d'agents tensioactifs anioniques, des mélanges d'agents tensioactifs anioniques et d'agents tensioactifs amphotères, cationiques ou non ioniques, des mélanges d'agents tensioactifs cationiques avec des agents tensioactifs non ioniques ou amphotères. Un mélange particulièrement préféré est un mélange comprenant au moins un agent tensioactif anionique et au moins un agent tensioactif amphotère.

La quantité d'agents tensioactifs anioniques va de 3 % à 40% en poids, rapportée au poids total de la composition cosmétique. Elle est de préférence comprise entre 5 % et 35 % en poids et, mieux encore, entre 8 % et 25 % en poids.

La quantité d'agents tensioactifs amphotères et/ou non ioniques, lorsqu'ils sont présents, va de préférence de 0,5 à 20 %, et en particulier de 1 à 15 % rapportée au poids total de la composition.

De manière générale, au sens de la présente invention, l'expression "silicone non aminée" désigne toute silicone ne contenant pas au moins une amine primaire, secondaire, tertiaire ou un groupement ammonium quaternaire.

Les silicones non aminées ayant une viscosité inférieure à 100.000 mm²/s utilisables conformément à l'invention sont en particulier des polyorganosiloxanes insolubles dans la composition et peuvent se présenter sous forme d'huiles, de cires, de résines. Les silicones ont de préférence une viscosité allant de 1000 à 100.000 cst et plus particulièrement de 10.000 à 80.000 cst.

Les organopolysiloxanes sont définis plus en détail dans l'ouvrage de Walter NOLL "Chemistry and Technology of Silicones" (1968) Académie Press.

On utilise de préférence des silicones non volatiles et plus particulièrement des polydialkylsiloxanes, des polydiarylsiloxanes, des polyalkylarylsiloxanes, des gommes et des résines de silicones, des polyorganosiloxanes modifiés par des groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyle ayant une viscosité allant de 100 à 100.000 mm²/s (cSt) à 25°C et de préférence 1000 à 100.000 mm²/s (cSt) et plus particulièrement de 10.000 à 80.000 mm²/s (cSt). La viscosité des silicones est par exemple mesurée à 25°C selon la norme ASTM 445 Appendice C.

Parmi ces polyalkylsiloxanes, on peut citer à titre non limitatif les produits commerciaux suivants :
- les huiles de la série MIRASIL commercialisées par la société RHODIA CHIMIE, telles que par exemple l'huile MIRASIL DM 500 ;
- les huiles de la série 200 de la société DOW CORNING telles que plus particulièrement la DC200 de viscosité 60 000 cSt ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHONE POULENC .

Dans cette classe de polydialkylsiloxanes, on peut également citer les produits commercialisés sous les dénominations "ABIL WAX 9800 et 9801" par la société GOLDSCHMIDT qui sont des polydialkyl (C₁-C₂₀) siloxanes.

Les polyalkylarylsiloxanes sont particulièrement choisis parmi les polydiméthyl méthylphénylsiloxanes, les polydiméthyl diphénylsiloxanes linéaires et/ou ramifiés de viscosité de 10 mm²/s à 10.000 mm²/s (cSt) à 25°C et plus particulièrement de 100 mm2/s à 5000 mm2/s.

Parmi ces polyalkylarylsiloxanes on peut citer à titre d'exemple les produits commercialisés sous les dénominations suivantes :
. les huiles SILBIONE de la série 70 641 de RHONE POULENC ;
. les huiles des séries RHODORSIL 70 633 et 763 de RHONE POULENC ;
. l'huile DOW CORNING 556 COSMETIC GRAD FLUID de DOW CORNING ;
. les silicones de la série PK de BAYER comme le produit PK20 ;
. les silicones des séries PN, PH de BAYER comme les produits PN1000 et PH1000;
. certaines huiles des séries SF de GENERAL ELECTRIC telles que SF 1023,
   SF 1154, SF 1250, SF 1265.

Les silicones organo modifiées utilisables conformément à l'invention sont des silicones telles que définies précédemment et comportant dans leur structure un ou plusieurs groupements organofonctionnels fixés par l'intermédiaire d'un radical hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET L 722, L 7500, L 77, L 711 de la société UNION CARBIDE et l'alkyl (C₁₂) méthicone copolyol commercialisée par la société DOW CORNING sous la dénomination Q2 5200 ;
- des groupements thiols comme les produits commercialisés sous les dénominations "GP 72 A" et "GP 71" de GENESEE ;
- des groupements alcoxylés comme le produit commercialisé sous la dénomination "SILICONE COPOLYMER F-755" par SWS SILICONES et ABIL WAX 2428, 2434 et 2440 par la société GOLDSCHMIDT ;
- des groupements hydroxylés comme les polyorganosiloxanes à fonction hydroxyalkyle décrits dans la demande de brevet français FR-A-85 16334
- des groupements acyloxyalkyle tels que par exemple les polyorganosiloxanes décrits dans le brevet US-A-4957732.
- des groupements anioniques du type carboxylique comme par exemple dans les produits décrits dans le brevet EP 186 507 de la société CHISSO CORPORATION, ou de type alkylcarboxyliques comme ceux présents dans le produit X-22-3701 E de la société SHIN-ETSU ; 2-hydroxyalkylsulfonate ; 2-hydroxyalkylthiosulfate tels que les produits commercialisés par la société GOLDSCHMIDT sous les dénominations "ABIL S201" et "ABIL S255".

Selon l'invention, on peut également utiliser des silicones comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans les demandes de brevet EP-A-412 704, EP-A-412 707, EP-A-640 105 et WO 95/00578, EP-A-582 152 et WO 93/23009 et les brevets US 4,693,935, US 4,728,571 et US 4,972,037. Ces polymères sont de préférence anioniques ou non ioniques.

De tels polymères sont par exemple les copolymères susceptibles d'être obtenus par polymérisation radicalaire à partir du mélange de monomères constitué par :
a) 50 à 90% en poids d'acrylate de tertiobutyle ;
b) 0 à 40% en poids d'acide acrylique ;
c) 5 à 40% en poids de macromère siliconé de formule : 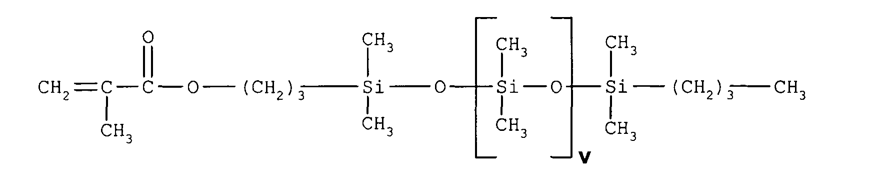 avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Selon l'invention, toutes les silicones peuvent également être utilisées sous forme d'émulsions, de nanoémulsions ou de micrémulsions.

Les polyorganosiloxanes particulièrement préférés conformément à l'invention sont :
- les silicones non volatiles choisies dans la famille des polydialkylsiloxanes à groupements terminaux triméthylsilyle telles que les huiles ayant une viscosité comprise entre 1000 et 100.000 mm²/s (cSt) à 25° C telles que les huiles de la séries DC200 de DOW CORNING en particulier l'huile de viscosité 60 000 mm²/s (cSt), des séries MIRASIL DM, les polydialkylsiloxanes à groupements terminaux diméthylsilanol tels que les diméthiconol ou les polyalkylarylsiloxanes tels que l'huile MIRASIL DPDM commercialisée par la société RHODIA CHIMIE ;

Selon l'invention, la ou les silicones non aminées peuvent représenter de 0,001 % à 20 % en poids, de préférence de 0,01 % à 10% en poids et plus particulièrement de 0,1 à 3% en poids par rapport au poids total de la composition finale.

Selon un mode préféré de l'invention, les compositions peuvent comprendre en outre au moins un polymère cationique différent du polymère filant selon l'invention.

Les polymères cationiques utilisables conformément à la présente invention peuvent être choisis parmi tous ceux déjà connus en soi comme améliorant les propriétés cosmétiques des cheveux, à savoir notamment ceux décrits dans la demande de brevet EP-A- 0 337 354 et dans les demandes de brevets français FR-A- 2 270 846, 2 383 660, 2 598 611, 2 470 596 et 2 519 863 et ayant une densité de charge cationique convenable.

De manière encore plus générale, au sens de la présente invention, l'expression "polymère cationique" désigne tout polymère contenant des groupements cationiques et/ou des groupements ionisables en groupements cationiques.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse molaire moyenne en nombre ou en poids comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus.

Les polymères du type polyamine, polyamidoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :
(1) les homopolymères ou copolymères dérivés d'esters ou d'amides acryliques ou méthacryliques et comportant au moins un des motifs de formules suivantes: 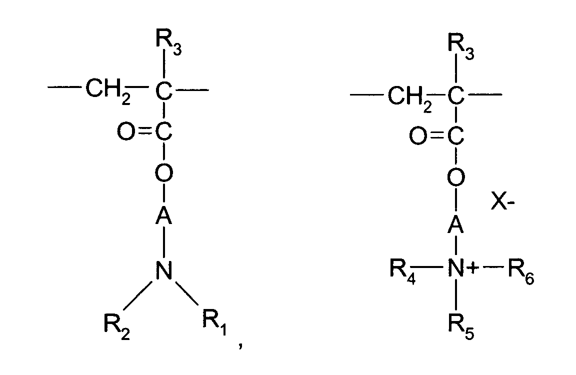 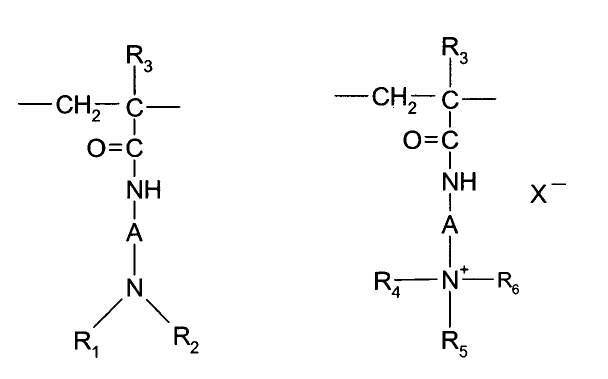 dans lesquelles:
   R₃ , identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂ , identiques ou différents, représentent hydrogène ou un groupe alkyle ayant de 1 à 6 atomes de carbone et de préférence méthyle ou éthyle;
   X désigne un anion dérivé d'un acide minéral ou organique tel que un anion méthosulfate ou un halogénure tel que chlorure ou bromure.

Les copolymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C1-C4), des acides acryliques ou méthacryliques ou leurs esters, des vinyllactames tels que la vinylpyrrolidone ou le vinylcaprolactame, des esters vinyliques.

Ainsi, parmi ces copolymères de la famille (1), on peut citer :
- les copolymères d'acrylamide et de diméthylaminoéthyl méthacrylate quaternisé au sulfate de diméthyle ou avec un halogénure de diméthyle tels que celui vendu sous la dénomination HERCOFLOC par la société HERCULES,
- les copolymères d'acrylamide et de chlorure de méthacryloyloxyéthyltriméthylammonium décrit par exemple dans la demande de brevet EP-A-080976 et vendus sous la dénomination BINA QUAT P 100 par la société CIBA GEIGY,
- le copolymères d'acrylamide et de méthosulfate de méthacryloyloxyéthyltriméthylammonium vendus sous la dénomination RETEN par la société HERCULES,
- les copolymères vinylpyrrolidone / acrylate ou méthacrylate de dialkylaminoalkyle quaternisés ou non. Ces polymères sont décrits en détail dans les brevets français 2.077.143 et 2.393.573,
- les terpolymères méthacrylate de diméthyl amino éthyle/vinylcaprolactame/ vinylpyrrolidone,
- les copolymères vinylpyrrolidone / méthacrylamidopropyl dimethylamine.
- et les copolymères vinylpyrrolidone / méthacrylamide de diméthylaminopropyle quaternisé.

(2) Les polysaccharides cationiques notamment les celluloses et les gommes de galactomannanes cationiques. Parmi les polysaccharides cationiques, on peut citer plus particulièrement les dérivés d'éthers de cellulose comportant des groupements ammonium quaternaires, les copolymères de cellulose cationiques ou les dérivés de cellulose greffés avec un monomère hydrosoluble d'ammonium quaternaire et les gommes de galactomannanes cationiques.

Les dérivés d'éthers de cellulose comportant des groupements ammonium quaternaires décrits dans le brevet français 1 492 597. Ces polymères sont également définis dans le dictionnaire CTFA comme des ammonium quaternaires d'hydroxyéthylcellulose ayant réagi avec un époxyde substitué par un groupement triméthylammonium.

Les copolymères de cellulose cationiques ou les dérivés de cellulose greffés avec un monomère hydrosoluble d'ammonium quaternaire, sont décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.

Les gommes de galactomannane cationiques sont décrites plus particulièrement dans les brevets US 3 589 578 et 4 031 307 en particulier les gommes de guar contenant des groupements cationiques trialkylammonium. On utilise par exemple des gommes de guar modifiées par un sel (par ex. chlorure) de 2,3-époxypropyl triméthylammonium.

(3) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361 ;

(4) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 ;

(5) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.

Parmi ces dérivés, on peut citer plus particulièrement les polymères acide adipique/diméthylaminohydroxypropyl/diéthylène triamine vendus sous la dénomination "CARTARETINE F, F4 ou F8" par la société SANDOZ.

(6) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.

Des polymères de ce type sont en particulier commercialisés sous la dénomination "HERCOSETT 57" par la société Hercules Inc. par la société HERCULES dans le cas du copolymère d'acide adipique/époxypropyl/diéthylènetriamine.

(7) les cyclopolymères d'alkyl diallyl amine ou de dialkyl diallyl ammonium tels que les homopolymères ou copolymères comportant comme constituant principal de la chaîne des motifs répondant aux formules (I) ou (I') : 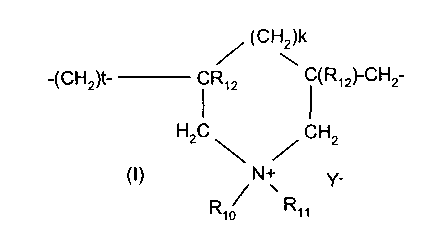 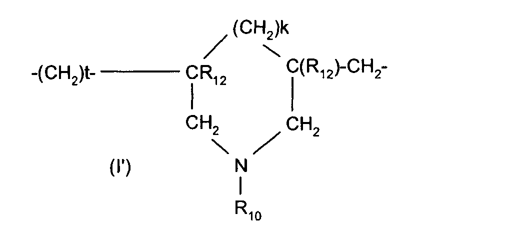 formules dans lesquelles k et t sont égaux à 0 ou 1, la somme k + t étant égale à 1 ; R₁₂ désigne un atome d'hydrogène ou un radical méthyle ; R₁₀ et R₁₁, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C1-C4) ou R₁₀ et R₁₁ peuvent désigner conjointement avec l'atome d'azote auquel ils sont rattachés, des groupement hétérocycliques, tels que pipéridinyle ou morpholinyle ; Y est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.

R₁₀ et R₁₁, indépendamment l'un de l'autre, désignent de préférence un groupement alkyle ayant de 1 à 4 atomes de carbone.

Parmi les polymères définis ci-dessus, on peut citer plus particulièrement l'homopolymère de chlorure de diméthyldiallylammonium vendu sous la dénomination "MERQUAT 100" par la société NALCO (et ses homologues de faibles masses molaires moyenne en poids) et les copolymères de chlorure de diallyldiméthylammonium et d'acrylamide .

(8) les polymères de diammonium quaternaire contenant des motifs récurrents répondant à la formule : 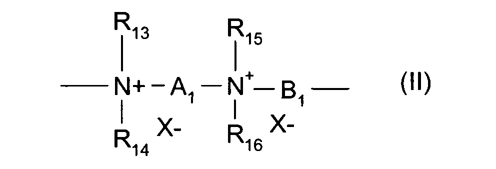 formule (II) dans laquelle :
R₁₃, R₁₄, R₁₅ et R₁₆, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote ou bien R₁₃, R₁₄, R₁₅ et R₁₆ représentent un radical alkyle en C1-C6 linéaire ou ramifié substitué par un groupement nitrile, ester, acyle, amide ou -CO-O-R₁₇-D ou -CO-NH-R₁₇-D où R₁₇ est un alkylène et D un groupement ammonium quaternaire ;
A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
X⁻ désigne un anion dérivé d'un acide minéral ou organique;
A1, R13 et R15 peuvent former avec les deux atomes d'azote auxquels ils sont rattachés un cycle pipérazinique ; en outre si A1 désigne un radical alkylène
ou hydroxyalkylène linéaire ou ramifié, saturé ou insaturé, B1 peut également désigner un groupement (CH2)n-CO-D-OC-(CH2)n-
dans lequel D désigne :
a) un reste de glycol de formule : -O-Z-O-, où Z désigne un radical hydrocarboné linéaire ou ramifié ou un groupement répondant à l'une des formules suivantes :

   -(CH2-CH2-O)x-CH2-CH2-

   -[CH2-CH(CH3)-O]y-CH2-CH(CH3)-

   où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine ;
c) un reste de diamine bis-primaire de formule : -NH-Y-NH-, où Y désigne un radical hydrocarboné linéaire ou ramifié, ou bien le radical bivalent

   -CH2-CH2-S-S-CH2-CH2- ;
d) un groupement uréylène de formule : -NH-CO-NH- ;

De préférence, X⁻ est un anion tel que le chlorure ou le bromure.

Ces polymères ont une masse molaire moyenne en nombre généralement comprise entre 1000 et 100000.

Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.

On peut utiliser plus particulièrement les polymères qui sont constitués de motifs récurrents répondant à la formule : 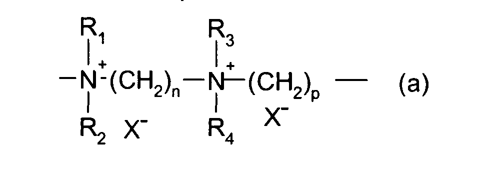 dans laquelle R₁, R₂, R₃ et R₄, identiques ou différents, désignent un radical alkyle ou hydroxyalkyle ayant de 1 à 4 atomes de carbone environ, n et p sont des nombres entiers variant de 2 à 20 environ et, X- est un anion dérivé d'un acide minéral ou organique.

Un composé de formule (a) particulièrement préféré est celui pour lequel R₁, R₂, R₃ et R₄, représentent un radical méthyle et n = 3, p = 6 et X = Cl, dénommé Hexadimethrine chloride selon la nomenclature INCl (CTFA).

(9) les polymères de polyammonium quaternaires comprenant des motifs de formule (III): 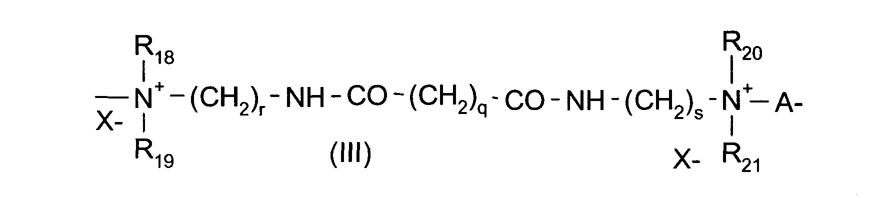 formule dans laquelle :
R₁₈, R₁₉, R₂₀ et R₂₁, identiques ou différents, représentent un atome d'hydrogène ou un radical méthyle, éthyle, propyle, β-hydroxyéthyle, β-hydroxypropyle ou -CH₂CH₂(OCH₂CH₂)ₚOH,
où p est égal à 0 ou à un nombre entier compris entre 1 et 6, sous réserve que R₁₈, R₁₉, R₂₀ et R₂₁ ne représentent pas simultanément un atome d'hydrogène,
r et s, identiques ou différents, sont des nombres entiers compris entre 1 et 6,
q est égal à 0 ou à un nombre entier compris entre 1 et 34,
X- désigne un anion tel qu'un halogènure,
A désigne un radical d'un dihalogénure ou représente de préférence -CH₂-CH₂-O-CH₂-CH₂-.

De tels composés sont notamment décrits dans la demande de brevet EP-A-122 324.

On peut par exemple citer parmi ceux-ci, les produits "Mirapol® A 15", "Mirapol® AD1 ", "Mirapol® AZ1" et "Mirapol® 175" vendus par la société Miranol.

(10) Les polymères quaternaires de vinylpyrrolidone et de vinylimidazole tels que par exemple les produits commercialisés sous les dénominations Luviquat® FC 905, FC 550 et FC 370 par la société B.A.S.F.

(11) Les polymères réticulés de sels de méthacryloyloxyalkyl(C1-C4) trialkyl(C1-C4)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine, notamment les chitosanes ou leurs sels ;
les sels utilisables sont en particulier l'acétate, le lactate, le glutamate, le gluconate ou le pyrrolidone-carboxylate de chitosane.

Parmi ces composés, on peut citer le chitosane ayant un taux de désacétylation de 90 % en poids, le pyrrolidone-carboxylate de chitosane commercialisé sous la dénomination KYTAMER® PC par la société AMERCHOL.

Parmi tous les polymères cationiques susceptibles d'être utilisés dans le cadre de la présente invention, on préfère mettre en oeuvre les cyclopolymères cationiques, en particulier les homopolymères ou copolymères de chlorure de diméthyldiallylammonium, vendus sous les dénominations « MERQUAT 100 », « MERQUAT 550 » et « MERQUAT S » par la société NALCO, les polymères quaternaires de vinylpyrrolidone et de vinylimidazole, les homopolymères ou copolymères réticulés de sels de méthacryloyloxyalkyl(C₁-C₄) trialkyl(C₁-C₄)ammonium, le pyrrolidone-carboxylate de chitosane et leurs mélanges.

Les polymères amphotères utilisables conformément à la présente invention peuvent être choisis parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes;

K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus plus particulièrement préférés sont choisis parmi les polymères suivants :
(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537. On peut également citer le copolymère acrylate de sodium / chlorure d'acrylamidopropyl trimethyl ammonium vendu sous la dénomination POLYQUART KE 3033 par la Société COGNIS.
   Le composé vinylique peut être également un sel de dialkyldiallylammonium tel que le sel (par exemple chlorure) de diméthyldiallylammonium. Les copolymères d'acide acrylique et de ce dernier monomère sont proposés sous les appellations MERQUAT 280, MERQUAT 295 par la société NALCO.
(2) Les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.

Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl-acrylamide, le N-tertiooctyl-acrylamide, le N-octylacrylamide, le N-décylacrylamide, le N-dodécylacrylamide ainsi que les méthacrylamides correspondants.

Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.

Les comonomères basiques préférés sont des méthacrylates d'aminoéthyle, de butyl aminoéthyle, de N,N'-diméthylaminoéthyle, de N-tertio-butylaminoéthyle.

On utilise particulièrement les copolymères dont la dénomination CTFA (4ème Ed., 1991) est Octylacrylamide/acrylates/butylaminoethylmethacrylate copolymer.

(3) Les polyaminoamides réticulés et alcoylés partiellement ou totalement dérivant de polyaminoamides de formule générale :

(̵CO―R₄―CO―Z)̵ (IV)

dans laquelle R₄ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
a) dans les proportions de 60 à 100 moles %, le radical 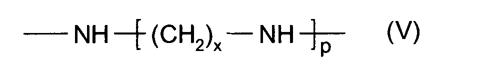 où x=2 et p=2 ou 3, ou bien x=3 et p=2
   ce radical dérivant de la diéthylène triamine, de la triéthylène tétraamine ou de la dipropylène triamine;
b) dans les proportions de 0 à 40 moles % le radical (V) ci-dessus, dans lequel x=2 et p=1 et qui dérive de l'éthylènediamine, ou le radical dérivant de la pipérazine : 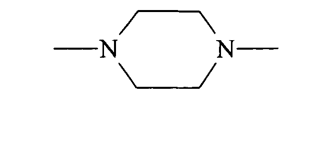
c) dans les proportions de 0 à 20 moles % le radical -NH-(CH₂)₆-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.

Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.

Les alcanes sultones utilisées dans l'alcoylation sont de préférence la propane ou la butane sultone, les sels des agents d'alcoylation sont de préférence les sels de sodium ou de potassium.

(4) Les polymères comportant des motifs zwittérioniques de formule : 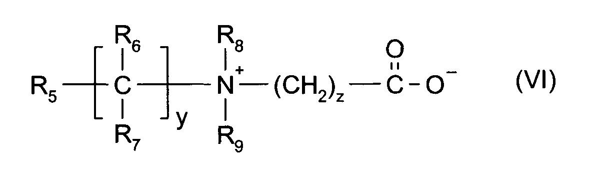 dans laquelle R₅ désigne un groupement insaturé polymérisable tel qu'un groupement acrylate, méthacrylate, acrylamide ou méthacrylamide, y et z représentent un nombre entier de 1 à 3, R₆ et R₇ représentent un atome d'hydrogène, méthyle, éthyle ou propyle, R₈ et R₉ représentent un atome d'hydrogène ou un radical alkyle de telle façon que la somme des atomes de carbone dans R₈ et R₉ ne dépasse pas 10.

Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.

A titre d'exemple, on peut citer le copolymère de méthacrylate de butyle/méthacrylate de diméthyl-carboxyméthylammonio-éthyl.

(5) les polymères dérivés du chitosane comportant des motifs monomères répondant aux formules (VII), (VIII) et (IX) suivantes : 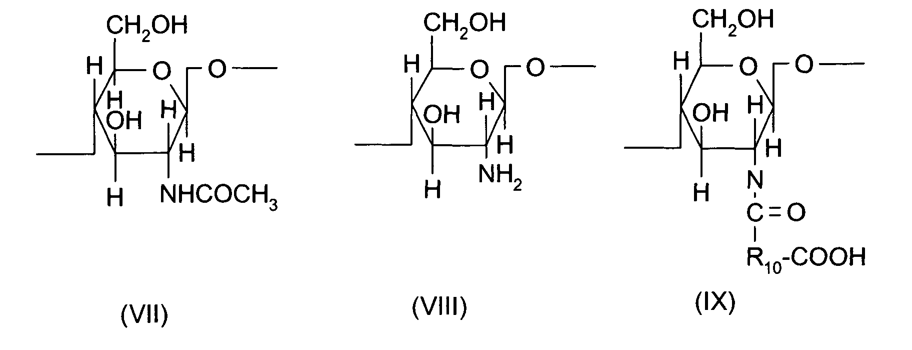 le motif (VII) étant présent dans des proportions comprises entre 0 et 30%, le motif (VIII) dans des proportions comprises entre 5 et 50% et le motif (IX) dans des proportions comprises entre 30 et 90%, étant entendu que dans ce motif (IX), R₁₀ représente un radical de formule : 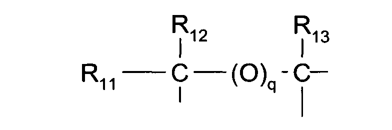 dans laquelle
si q=0, R₁₁, R₁₂ et R₁₃, identiques ou différents, représentent chacun un atome d'hydrogène, un reste méthyle, hydroxyle, acétoxy ou amino, un reste monoalcoylamine ou un reste dialcoylamine éventuellement interrompus par un
ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux R₁₁, R₁₂ et R₁₃ étant dans ce cas un atome d'hydrogène ;
ou si q=1, R₁₁, R₁₂ et R₁₃ représentent chacun un atome d'hydrogène, ainsi que les sels formés par ces composés avec des bases ou des acides.

(6) Les polymères dérivés de la N-carboxyalkylation du chitosane comme le N-carboxyméthyl chitosane ou le N-carboxybutyl chitosane.

(7) Les polymères répondant à la formule générale (X) tels que ceux décrits par exemple dans le brevet français 1 400 366 : 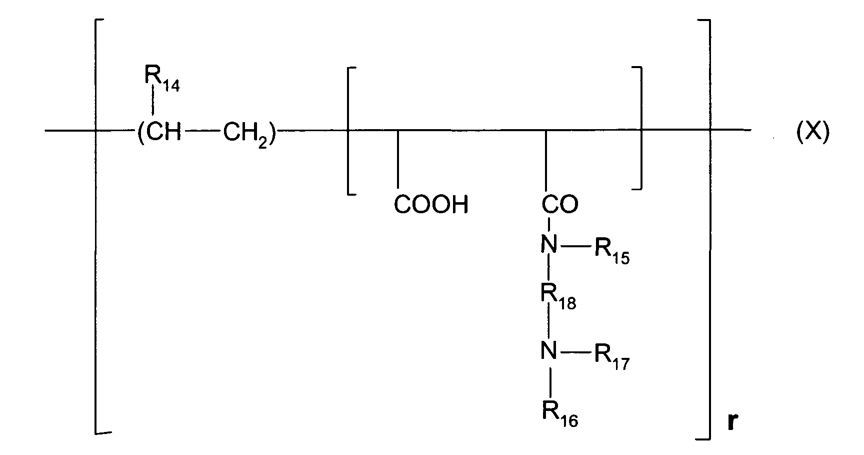 dans laquelle R₁₄ représente un atome d'hydrogène, un radical CH₃O, CH₃CH₂O, phényle, R₁₅ désigne l'hydrogène ou un radical alkyle inférieur tel que méthyle, éthyle, R₁₆ désigne l'hydrogène ou un radical alkyle inférieur tel que méthyle, éthyle, R₁₇ désigne un radical alkyle inférieur tel que méthyle, éthyle ou un radical répondant à la formule : -R₁₈-N(R₁₆)₂, R₁₈ représentant un groupement -CH₂-CH₂- , -CH₂-CH₂-CH₂- , -CH₂-CH(CH₃)- , R₁₆ ayant les significations mentionnées ci-dessus,
ainsi que les homologues supérieurs de ces radicaux et contenant jusqu'à 6 atomes de carbone.

(8) Des polymères amphotères du type -D-X-D-X- choisis parmi:
a) les polymères obtenus par action de l'acide chloracétique ou le chloracétate de sodium sur les composés comportant au moins un motif de formule :

   -D-X-D-X-D- (XI)

   où D désigne un radical 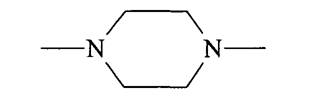 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;
b) les polymères de formule :

   -D-X-D-X- (XII)

   où D désigne un radical 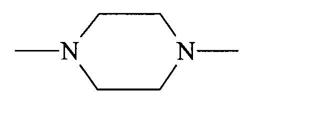 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.

(9) Les copolymères alkyl(C₁-C₅)vinyléther / anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Les polymères amphotères particulièrement préférés selon l'invention sont ceux de la famille (1 ).

De préférence, la composition selon l'invention peut contenir un ou plusieurs des polymères cationiques ou amphotères tels que décrits ci-dessus.
Selon l'invention, le ou les polymères cationiques ou amphotères peuvent représenter de 0,001 % à 20 % en poids, de préférence de 0,01 % à 10% en poids et plus particulièrement de 0,02 à 5% en poids par rapport au poids total de la composition finale.

Selon un mode particulier de l'invention, les compositions peuvent comprendre en outre au moins une silicone aminée.

Par milieu aqueux cosmétiquement acceptable, on entend un milieu compatible avec les matières kératiniques telles que notamment la peau, les cils, et les cheveux.

Le milieu aqueux cosmétiquement acceptable peut être constitué uniquement par de l'eau ou par un mélange d'eau et d'un solvant cosmétiquement acceptable tel qu'un alcool inférieur en C₁-C₄, tel que l'éthanol, l'isopropanol, le tertio-butanol, le n-butanol ; les alkylèneglycols comme le propylèneglycol, les éthers de polyol et leurs mélanges.

De préférence, la composition comprend de 50 à 95 % en poids d'eau par rapport au poids total de la composition.

Les compositions de lavage selon l'invention présentent un pH final allant généralement de 3 à 10. De préférence, ce pH est compris entre 5 et 8. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide, de préférence un acide carboxylique tel que par exemple l'acide citrique.

Les compositions conformes à l'invention peuvent contenir en plus de l'association définie ci-dessus des agents régulateurs de viscosité tels que des électrolytes, ou des agents épaississants (associatifs ou non associatifs). On peut citer en particulier le chlorure de sodium, le xylène sulfonate de sodium, les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit commercialisé sous la dénomination "AMINOL A15" par la société CHEM Y, les acides polyacryliques réticulés et les copolymères d'acide acrylique tels que les copolymères d'acide acrylique et d'acrylates d'alkyle en C₁₀-C₃₀ réticulés. Ces agents régulateurs de viscosité sont utilisés dans les compositions selon l'invention dans des proportions pouvant aller jusqu'à 10 % en poids par rapport au poids total de la composition.

Les compositions conformes à l'invention peuvent également contenir de préférence jusqu'à 5 % d'agents nacrants ou opacifiants bien connus dans l'état de la technique tels que par exemple les alcools gras supérieurs à C16, les dérivés acylés à chaîne grasse tels que les monostéarates ou distéarates d'éthylène glycol ou de polyéthylèneglycol, les éthers à chaînes grasses (C10-C30) tels que par exemple le distéaryléther ou le 1-(hexadécyloxy)-2-octadécanol.

Les compositions conformes à l'invention peuvent éventuellement contenir en outre au moins un additif choisi parmi les synergistes de mousses tels que des 1,2-alcanediols en C₁₀-C₁₈ ou des alcanolamides gras dérivés de mono ou de diéthanolamine, les filtres solaires siliconés ou non, les polymères anioniques ou non ioniques, les tensioactifs cationiques, les protéines, les hydrolysats de protéines, les céramides, les pseudocéramides, les acides gras à chaînes linéaires ou ramifiées en C₁₂-C₄₀ tels que l'acide méthyl-18 eicosanoique, les hydroxyacides, les vitamines, les provitamines tels que le panthénol, les huiles animales, minérales ou de synthèse et tout autre additif classiquement utilisé dans le domaine cosmétique qui n'affecte pas les propriétés des compositions selon l'invention.

Les compositions de lavage selon l'invention peuvent bien entendu contenir en outre tous les adjuvants usuels rencontrés dans le domaine des shampooings, comme par exemple des parfums, des agents conservateurs, des sequestrants, des adoucissants, des colorants, des agents hydratants, des agents anti-pélliculaires ou anti-séborrhéiques, et autres.

Bien entendu, l'homme de l'art veillera à choisir ce ou ces éventuels composés complémentaires et/ou leurs quantités de manière telle que les propriétés avantageuses attachées intrinsèquement à l'association conforme à l'invention ne soient pas, ou substantiellement pas, altérées par la ou les adjonctions envisagées.

Ces compositions peuvent se présenter sous la forme de liquides, éventuellement épaissis, de crèmes ou de gel et elles conviennent principalement au lavage, et éventuellement au soin et/ou le coiffage des cheveux.

La présente invention concerne également un procédé de traitement cosmétique des matières kératiniques qui consiste à appliquer une quantité efficace d'une composition telle que décrite ci-dessus, sur les matières kératiniques, à effectuer un rinçage après un éventuel temps de pose.

Selon un mode de réalisation préféré de l'invention, la composition peut être utilisée comme shampoing.

Lorsque les compositions conformes à l'invention sont mises en oeuvre comme des shampooings classiques, elles sont simplement appliquées sur cheveux mouillés et la mousse générée par massage ou friction avec les mains est ensuite éliminée, après un éventuel temps de pause, par rinçage à l'eau, l'opération pouvant être répétée une ou plusieurs fois.

Des exemples concrets, mais nullement limitatifs, illustrant l'invention vont maintenant être donnés.

### EXEMPLE 1

On a réalisé une composition de shampooing conforme à l'invention :
- Lauryléthersulfate de sodium (C12/C14 à 70/30) à 2,2 moles d'oxyde d'éthylène à 26%MA (MA = matière active) 12 g MA
- Polydiméthylsiloxane de viscosité 60.000 cSt (Fluid DC60.000 cst de DOW CORNING) 1,5 g
- Copolymère d'acrylamide et de diméthylamino éthyl quaternisé par du chlorure de benzyle commercialisé sous la dénomination ULTIMER par la société ONDEO 0,8 g
- Chlorure de sodium 2 g
- Acide citrique ou NaOH qs pH 6,5
- Eau qsp 100 g

Les cheveux traités avec ce shampooing présentent de bonnes propriétés de coiffage et de corporisation.

### EXEMPLES 2 à 5

| | 2 | 3 | 4 | 5 |
|---|---|---|---|---|
| Copolymère (90/10 en mole) d'acrylamide et de diméthylamino éthyl quaternisé par du chlorure de benzyle (PM>5.10⁶) en dispersion dans une solution aqueuse saline concentrée (ULTIMER de ONDEO) | 1 % MA | 0.8% MA | - | 0.5% MA |
| Copolymère (10/90 en moles) de diméthyl Aminoéthyl Acrylate quaternisé par du chlorure de méthyle et d'acrylamide (PM>5.10⁶) en dispersion dans une solution aqueuse saline concentrée | - | - | 0.5%MA | - |
| Lauryl ether sulfate de sodium 2.2 OE | 12.5% MA | 12% MA | 7.5% MA | 10% MA |
| Coco Amido propyl bétaine | 2.5% MA | 1.5% MA | 2.5% MA | 2% MA |
| Dimethicone (DC 200 Fluid 60 000 cSt de Dow Corning) | 1% | 1.5% | - | - |
| Dimethicone (DC 200 Fluid 1000 cSt de Dow Corning) | - | - | 1% | - |
| Phenyl Trimethicone (Belsil PDM 1000 de Wacker) | - | - | - | 0.8% |
| Ethanol | - | 1% | - | - |
| NaCl | - | - | - | 2% |
| Eau qsp | 100 % | 100 % | 100 % | 100 % |

Les cheveux traités avec ces compositions présentent les mêmes propriétés que ceux traités avec la composition de l'exemple 1.

## Revendications

1. Composition de lavage des matières kératiniques **caractérisée en ce qu'**elle comprend, dans un milieu aqueux cosmétiquement acceptable, au moins une silicone non aminée ayant une viscosité inférieure à 100.000 mm²/s (cSt), au moins un tensioactif détergent anionique, non ionique ou amphotère et au moins un polymère présentant un pouvoir filant supérieur à 5 cm.

2. Composition selon la revendication 1, **caractérisée par le fait que** les tensioactifs détergents sont choisis parmi les tensioactifs anioniques.

3. Composition selon la revendication 1 ou 2, **caractérisée par le fait que** ledit tensioactif est présent à une teneur pondérale comprise entre 4 % et 50 % par rapport au poids total de la composition.

4. Composition selon la revendication 3, **caractérisée par le fait que** ladite teneur est comprise entre 8 % et 25 %.

5. Composition selon l'une quelconque des revendications 1 à 4, **caractérisée en ce que** le poylmère filant est choisi parmi:
soit (a1) une dispersion de particules d'un polymère cationique hydrosoluble ayant une masse moléculaire moyenne en poids supérieure à 10⁶ dans une solution aqueuse saline, obtenu par polymérisation radicalaire hétérogène de monomères hydrosolubles avec précipitation du polymère formé, ou soit (a2) une solution aqueuse d'un polymère cationique hydrosoluble ayant une masse moléculaire moyenne en poids supérieure à 10⁶, obtenu par polymérisation radicalaire hétérogène de monomères hydrosolubles avec précipitation du polymère formé.

6. Composition selon la revendication 5, **caractérisé par le fait que** le ou les monomères hydrosolubles sont choisis parmi les monomères cationiques, et/ou non-ioniques comportant au moins une double liaison éthylénique ou un mélange de ceux-ci.

7. Composition selon la revendication 6, **caractérisé par le fait que** les monomères non-ioniques sont choisis parmi l'acrylamide, le méthacrylamide, le N-vinylformamide, le N-vinylacétonamide, l'acrylate d'hydroxypropyle et le méthacrylate d'hydroxypropyle.

8. Composition selon la revendication 6, **caractérisé par le fait que** les monomères cationiques sont choisis parmi les sels de di(alkyle en C₁-C₄)-diallylammonium et les composés de formule (I) 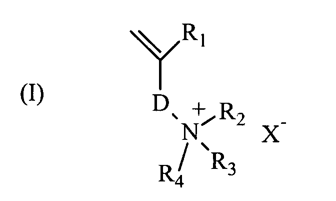 dans laquelle
R₁ représente un atome d'hydrogène ou un groupe méthyle,
R₂ et R₃, identiques ou différents, représentent chacun un atome d'hydrogène ou un groupe alkyle en C₁₋₄ linéaire ou ramifié,
R₄ représente un atome d'hydrogène, un groupe alkyle en C₁₋₄ linéaire ou ramifié ou un groupe aryle,
D représente le motif suivant 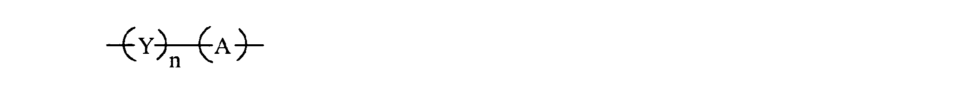 dans lequel Y représente une fonction amide, ester, uréthane ou urée,
A représente un groupe alkyène en C₁₋₁₀ linéaire, ramifié ou cyclique, pouvant être substitué ou interrompu par un cycle aromatique ou hétéroaromatique divalent, ou pouvant être interrompu par un hétéroatome choisi parmi O, N, S et P, et pouvant comporter une fonction cétone, amide, ester, uréthane ou urée,
n vaut 0 ou 1, et
X⁻ représente un contre-ion anionique tel qu'un ion halogénure ou sulfate.

9. Composition selon l'une quelconque des revendications 5 à 8, **caractérisé par le fait que** le polymère cationique hydrosoluble est polymérisé à partir d'au moins un monomère cationique de formule (I).

10. Composition selon la revendication 9, **caractérisé par le fait que** le polymère cationique hydrosoluble est polymérisé à partir d'un mélange de monomères comprenant de 0 à 95,5 % en moles d'acrylamide et de 4,5 à 100 % en moles d'au moins un monomère cationique de formule (I).

11. Composition selon l'une quelconque des revendications 9 ou 10, **caractérisé par le fait que** le polymère cationique hydrosoluble est polymérisé à partir d'un mélange de monomères constitué d'acrylamide et d'un monomère cationique de formule (I), le nombre de moles du monomère cationique de formule (I) étant supérieur au nombre de moles d'acrylamide.

12. Composition selon l'une quelconque des revendications 9 à 11, **caractérisé par le fait que** le polymère cationique hydrosoluble est polymérisé à partir d'un mélange de monomères constitué de 10 % en moles de chlorure d'acryloyloxyéthyldiméthylbenzyl-ammonium et de 90 % en moles d'acrylamide.

13. Composition selon l'une quelconque des revendications 9 à 12, **caractérisé par le fait que** le polymère cationique hydrosoluble est polymérisé à partir d'un mélange de monomères constitué de 30 % en moles de chlorure d'acryloyloxytriméthylammonium, de 50 % en moles de chlorure d'acryloyloxyéthyldiméthybenzylammonium et de 20 % en moles d'acrylamide.

14. Composition selon l'une quelconque des revendications 9 à 13, **caractérisé par le fait que** le polymère cationique hydrosoluble est polymérisé à partir de 10 % en moles de chlorure d'acryloyloxyéthyltriméthylammonium et de 90 % en moles d'acrylamide.

15. Composition selon l'une quelconque des revendications 9 à 11, **caractérisé par le fait que** le polymère cationique hydrosoluble est polymérisé à partir de 30 % en moles de chlorure de diallyldiméthylammonium et de 70 % en moles d'acrylamide.

16. Composition selon l'une quelconque des revendications 5 à 15, **caractérisé par le fait que** la concentration des particules de polymère cationique hydrosoluble en dispersion dans une solution aqueuse saline dans (a1) va de 0,01 à 20 % en poids.

17. Composition selon l'une quelconque des revendications 5 à 16, **caractérisé par le fait que** la concentration du polymère hydrosoluble en solution dans (a2) va de 0,01 à 20 % en poids.

18. Composition selon l'une quelconque des revendications 5 à 17, **caractérisé par le fait que** la solution aqueuse saline dans (a1 ) ou (a2) contient au moins un sel anionique.

19. Composition selon la revendication 18, **caractérisé par le fait que** le sel anionique est choisi parmi le sulfate d'ammonium, l'hydrogénosulfate d'ammonium, le chlorure d'ammonium, le sulfate de sodium, l'hydrogénosulfate de sodium, le sulfate de magnésium, l'hydrogénosulfate de magnésium, le sulfate d'aluminium, l'hydrogénosulfate d'aluminium.

20. Composition selon l'une quelconque des revendications précédentes, **caractérisée par le fait que** les polymères filants sont présents dans les composition de l'invention à une concentration va de 0,01 et 10% en poids, de préférence de 0,05 et 5% par rapport au poids total de la composition.

21. Composition selon l'une quelconque des revendications 1 à 20, **caractérisée par le fait que** les silicones non aminées ont une viscosité allant de 1000 à 100.000 mm²/s (cSt) et plus particulièrement de 10.000 à 80.000 mm²/s (cSt).

22. Composition selon l'une quelconque des revendications 1 à 21, **caractérisée par le fait que** les silicones non aminées sont des polyorganosiloxanes non volatils choisis parmi les polyalkylsiloxanes, les polyarylsiloxanes, les polyalkylarylsiloxanes, les résines de silicones, les polyorganosiloxanes modifiés par des groupements organofonctionnels ainsi que leurs mélanges.

23. Composition selon la revendication 22, **caractérisée par le fait que**
(a) les polydialkylsiloxanes sont choisis parmi :
- les polydiméthylsiloxanes à groupements terminaux triméthylsilyle ;
- les polydiméthylsiloxanes à groupements terminaux diméthylsilanol ;
- les polyalkyl(C₁-C₂₀)siloxanes ;
(b) les polyalkylarylsiloxanes sont choisis parmi :
- les polydiméthylméthylphénylsiloxanes, les polydiméthyldiphényl siloxanes linéaires et/ou ramifiés de viscosité comprise entre 1.10⁻⁵ et 5.10⁻²m²/s à 25°C ;
(c) les silicones organo modifiées sont choisies parmi les silicones comportant dans leur structure un ou plusieurs groupements organofonctionnels fixés par l'intermédiaire d'un radical hydrocarboné.

24. Composition selon l'une quelconque des revendications 22 ou 23, **caractérisée par le fait que** les silicones organomodifiées sont choisies parmi les polyorganosiloxanes comportant :
a) des groupements polyéthylèneoxy et/ou polypropylèneoxy ;
b) des groupements thiols ;
c) des groupements alcoxylés,
d) des groupements hydroxylés,
e) des groupements acyloxyalkyle,
f) des groupements alkyl carboxyliques,
g) des groupements 2-hydroxyalkylsulfonates,
h) des groupements 2-hydroxyalkylthiosulfonates,

25. Composition selon l'une quelconque des revendications 1 à 24, **caractérisée par le fait que** les silicones sont choisies parmi les polydialkylsiloxanes à groupements terminaux triméthylsilyle, les polydialkylsiloxanes à groupements terminaux diméthylsilanol, les polyalkylarylsiloxanes.

26. Composition selon l'une quelconque des revendications 1 à 25, **caractérisée par le fait que** les silicones non aminées sont choisies parmi les polyorganosiloxanes insolubles dans la composition.

27. Composition selon l'une quelconque des revendications précédentes, **caractérisée en ce que** la silicone non aminée est présente à une concentration allant de 0,001 % à 20 % en poids par rapport au poids total de la composition, de préférence de 0,01 % à 10 % en poids.

28. Composition selon l'une quelconque des revendications précédentes, **caractérisée par le fait que** la composition comprend en outre un polymère cationique.

29. Composition selon l'une quelconque des revendications précédentes, **caractérisée par le fait que** la composition comprend en outre une silicone aminée.

30. Composition selon l'une quelconque des revendications précédentes, **caractérisée par le fait qu'**elle présente un pH allant de 3 à 10.

31. Utilisation en cosmétique d'une composition telle définie à l'une quelconque des revendications précédentes pour le nettoyage et/ou le soin et/ou le conditionnement et/ou le coiffage des cheveux.
